# EUROPEAN PATENT APPLICATION

(11) **EP 4 542 575 A1**
(43) Date of publication of application: **23.04.2025**
(21) Application number: 23843421.1
(22) Date of filing: 21.07.2023
(51) Int. Cl.: G16H 50/50, G16H 50/20, G16H 50/70, G16H 10/60, A61B 5/00, A61B 5/0245

(54) **METHOD, PROGRAM, AND DEVICE FOR PREDICTING HEALTH STATUS USING ELECTROCARDIOGRAM**

(30) Priority: 22.07.2022 KR 20220090767; 18.07.2023 KR 20230093176
(71) Applicant: Medical AI Co., Ltd., Seoul 06180 (KR)
(72) Inventor: KWON, Joonmyoung, Seoul 06180 (KR); LEE, Minsung, Seoul 06180 (KR); SON, Jeongmin, Seoul 06180 (KR); KANG, Sora, Seoul 06180 (KR)
(74) Representative: Patentanwaltskanzlei Matschnig & Forsthuber OG
(86) International application number: PCT/KR2023/010539
(87) International publication number: WO 2024/019575

(57) **Abstract**

According to an embodiment of the present disclosure, a method, program, and device for predicting a health state using an electrocardiogram, performed by a computing device, are disclosed. The method may include: acquiring electrocardiogram data and cardiac arrest data including whether cardiac arrest occurs in a subject whose electrocardiogram data is measured and a cardiac arrest occurrence time of the subject; analyzing a missing value of the electrocardiogram data or the cardiac arrest data to extract valid data from the electrocardiogram data; and labeling the extracted valid data based on whether the cardiac arrest occurs and the cardiac arrest occurrence time to generate training data.

## Description

### [Technical Field]

The present disclosure relates to an artificial intelligence technology in the field of medicine, and more particularly, to a method for training an artificial intelligence model based on results of filtering electrocardiogram data reflecting a cardiac arrest state, and predicting a health state using the trained model.

### [Background Art]

An electrocardiogram is a test that records electrical activity of a heart. An electrocardiogram is a relatively simple and cost-effective test method of checking a health state of a heart, and plays an important role in the early diagnosis and management of heart disease. For example, an electrocardiogram signal measured through an electrocardiogram test may be used to check whether each part of the heart is functioning normally, whether a size and location of the heart are normal, and whether there is damage to a heart muscle, etc. **In** addition, an electrocardiogram signal may be used to diagnose various heart-related problems and predict a person's health state based on the check.

Meanwhile, as artificial intelligence (AI) technology develops, attempts to predict a person's health state based on an electrocardiogram using artificial intelligence technology are increasing. As the artificial intelligence technology is introduced, the most important part is securing appropriate data necessary for the artificial intelligence learning. Since the electrocardiogram records heart activity, cardiac arrest has a significant impact on the interpretation of the electrocardiogram, but most conventional technologies often do not reflect the occurrence and status of cardiac arrest in data secured for the artificial intelligence learning.

### [Disclosure]

### [Technical Problem]

The present disclosure provides a method of training an artificial intelligence model based on results of filtering electrocardiogram data reflecting cardiac arrest status and predicting a health state with the trained model.

However, the problems to be solved by the present disclosure are not limited to the problems described above, and other problems that are not mentioned may be clearly understood based on the description below.

### [Technical Solution]

According to an embodiment of the present disclosure, a method of predicting a health state using an electrocardiogram, performed by a computing device is disclosed. The method may include: acquiring electrocardiogram data and cardiac arrest data including whether cardiac arrest occurs in a subject whose electrocardiogram data is measured and a cardiac arrest occurrence time of the subject; analyzing a missing value of the electrocardiogram data or the cardiac arrest data to extract valid data from the electrocardiogram data; and labeling the extracted valid data based on whether the cardiac arrest occurs and the cardiac arrest occurrence time to generate training data.

The analyzing of the missing value of the electrocardiogram data or the cardiac arrest data to extract the valid data from the electrocardiogram data may include: analyzing the cardiac arrest occurrence time recorded in the cardiac arrest data and clinical evidence regarding cardiac arrest to determine a cutoff time for filtering the electrocardiogram data; and filtering the electrocardiogram data of the subject who suffers from cardiac arrest within the cutoff time among the acquired electrocardiogram data to extract the valid data.

The analyzing of the cardiac arrest occurrence time recorded in the cardiac arrest data and the clinical evidence regarding the cardiac arrest to determine the cutoff time for filtering the electrocardiogram data may include determining, as the cutoff time, one of a first candidate time derived based on clinical determination on a pattern of change in the electrocardiogram that occur before the cardiac arrest occurs, a second candidate time clinically determined as a time at which treatment is possible to perform to prevent the cardiac arrest when the cardiac arrest is predicted to occur, and a third candidate time corresponding to an error between the cardiac arrest occurrence time recorded in the cardiac arrest data and an actual cardiac arrest occurrence time of the subject whose electrocardiogram data is measured.

The cutoff time may be determined as the largest value among the first candidate time, the second candidate time, or the third candidate time.

The labeling of the extracted valid data based on whether the cardiac arrest occurs and the cardiac arrest occurrence time to generate the training data may include: labeling, as a cardiac arrest group, data of the subject who suffers from the cardiac arrest within a preset time after the cutoff time in the valid data; and labeling, as a normal group, data of the subject who does not suffer from the cardiac arrest or die within the preset time after the cutoff time in the valid data.

The analyzing of the missing value of the electrocardiogram data or the cardiac arrest data to extract the valid data from the electrocardiogram data may include excluding the electrocardiogram data in which one or more of N derivations of the electrocardiogram data themselves are missing from the valid data when one or more of the N derivations of the electrocardiogram data themselves miss.

The analyzing of the missing value of the electrocardiogram data or the cardiac arrest data to extract the valid data from the electrocardiogram data may include excluding, from the valid data, the electrocardiogram data including a derivation in which the missing value exists for a predetermined period of time or longer among the N derivations when there is the derivation in which the missing value exists for the predetermined period of time or longer among the N derivations of the electrocardiogram data.

The method may further include inputting the training data to a deep learning model to train the deep learning model so that the deep learning model calculates the possibility of occurrence of the cardiac arrest.

According to another embodiment of the present disclosure, a method of predicting a health state using an electrocardiogram, performed by a computing device is disclosed. The method may include: acquiring first electrocardiogram data; and inputting the first electrocardiogram data to a pre-trained deep learning model to predict the health state of a subject whose electrocardiogram data is measured. The deep learning model may be trained based on training data generated by labeling valid data extracted from second electrocardiogram data based on whether cardiac arrest occurs and a cardiac arrest occurrence time. The valid data may be extracted from the second electrocardiogram data based on results of analyzing a missing value of the second electrocardiogram data or cardiac arrest data including whether the cardiac arrest occurs in a subject whose second electrocardiogram data is measured and a cardiac arrest occurrence time of the subject.

The prediction result derived by inputting the first electrocardiogram to the pre-trained deep learning model may include a score value indicating the health state of the subject whose electrocardiogram data is measured. The score value may indicate a healthy state as it approaches a first threshold value, and indicate an unhealthy state as it approaches a second threshold value.

According to another embodiment of the present disclosure, a computer program stored in a computer-readable storage medium is disclosed. The computer program performs operations for predicting a health state using an electrocardiogram when executed on one or more processors. The operations may include: acquiring electrocardiogram data and cardiac arrest data including whether cardiac arrest occurs in a subject whose electrocardiogram data is measured and a cardiac arrest occurrence time of the subject; analyzing a missing value of the electrocardiogram data or the cardiac arrest data to extract valid data from the electrocardiogram data; and labeling the extracted valid data based on whether the cardiac arrest occurs and the cardiac arrest occurrence time to generate training data.

According to another embodiment of the present disclosure, a computing device for predicting a health state using an electrocardiogram is disclosed. The device may include: a processor including at least one core; a memory including program codes executable in the processor; and a network unit acquiring electrocardiogram data and cardiac arrest data including whether cardiac arrest occurs in a subject whose electrocardiogram data is measured and a cardiac arrest occurrence time of the subject. The processor may analyze a missing value of the electrocardiogram data or the cardiac arrest data to extract valid data from the electrocardiogram data, and label the extracted valid data based on whether the cardiac arrest occurs and the cardiac arrest occurrence time to generate training data.

### [Advantageous Effects]

The present disclosure may provide the method of training an artificial intelligence model based on the results of filtering the electrocardiogram data reflecting the cardiac arrest status and predicting the health state with the trained model.

### [Description of Drawings]

FIG. 1 is a block diagram of a computing device according to an embodiment of the present disclosure.
FIG. 2 is a block diagram illustrating a process of extracting valid data from electrocardiogram data according to an embodiment of the present disclosure.
FIG. 3 is a block diagram illustrating a process of training a deep learning model based on valid data according to an embodiment of the present disclosure.
FIG. 4 is a flowchart illustrating a method of predicting a health state using an electrocardiogram according to an embodiment of the present disclosure.
FIG. 5 is a flowchart illustrating a method of predicting a health state using an electrocardiogram according to an alternative embodiment of the present disclosure.

### [Best Mode]

Hereinafter, embodiments of the present disclosure will be described in detail with reference to the attached drawings so that those skilled in the art can easily implement the present disclosure. The embodiments presented in the present disclosure are provided so that those skilled in the art may utilize or implement contents of the present disclosure. Accordingly, various modifications to the embodiments of the present disclosure will be apparent to those skilled in the art. That is, the present disclosure may be implemented in various different forms, and is not limited to embodiments described below.

Throughout the specification of the present disclosure, the same or similar drawing symbols refer to the same or similar components. In addition, in order to clearly describe the present disclosure, drawing symbols of parts that are not related to the description of the present disclosure may be omitted in the drawings.

The term "or" used in the present disclosure is intended to mean an inclusive "or," not an exclusive "or." That is, unless otherwise specified in the present disclosure or the meaning is clear from the context, "X uses A or B" is intended to mean one of the natural inclusive substitutions. For example, unless otherwise specified in the present disclosure or the meaning is clear from the context, "X utilizes A or B" may be construed as one of X utilizing A, X utilizing B, or X utilizing both A and B."

The term "and/or" used in the present disclosure should be understood to refer to and include all possible combinations of one or more of the related concepts listed.

The terms "include" and/or "including" used in the present disclosure should be understood to mean that specific features and/or components are present. However, the terms "include" and/or "including" should be understood as not excluding the presence or addition of one or more other features, components and/or groups thereof.

In addition, unless otherwise specified in the present disclosure or the context is clear to indicate a singular form, the singular form should generally be construed to include "one or more."

The term "N^{th} (N is a natural number)" used in the present disclosure may be understood as an expression used to mutually distinguish components of the present disclosure according to a predetermined standard such as a functional perspective, a structural perspective, or convenience of description. For example, components performing different functional roles in the present disclosure may be distinguished as a first component or a second component. However, components that are substantially the same within the technical idea of the present disclosure but should be distinguished for convenience of description may also be distinguished as a first component or a second component.

The term "acquisition" used in the present disclosure may be understood to mean not only receiving data through a wired or wireless communication network with an external device or system, but also generating data in an on-device form.

Meanwhile, the term "module" or "unit" used in the present disclosure may be understood as a term referring to an independent functional unit that processes computing resources, such as a computer-related entity, firmware, software or a part thereof, hardware or a part thereof, or a combination of software and hardware. **In** this case, the "module" or "unit" may be a unit composed of a single element or may be a unit expressed as a combination or set of multiple elements. For example, as a narrow concept, "module" or "unit" may refer to hardware elements of a computing device or a set thereof, an application program that performs a specific function of software, a processing process implemented through software execution, a set of instructions for program execution, etc. **In** addition, as a broad concept, "module" or "unit" may refer to a computing device itself that constitutes a system, an application that is executed on the computing device, etc. However, the above-described concept is only an example, and the concept of "module" or "unit" may be variously defined within a category understandable to those skilled in the art based on the content of the present disclosure.

The term "model" used in the present disclosure may be understood as a system implemented using mathematical concepts and language to solve a specific problem, a set of software units to solve a specific problem, or an abstraction model of a processing process to solve a specific problem. For example, a neural network "model" may be the entire system implemented as a neural network that has a problem-solving ability through learning. **In** this case, the neural network may obtain its problem-solving ability by optimizing parameters connecting nodes or neurons through learning. The neural network "model" may include a single neural network or may include a neural network set that combines a plurality of neural networks.

The description of the above-described terms is intended to help understanding of the present disclosure. Therefore, when the above-described terms are not explicitly described as matters limiting the contents of the present disclosure, it should be noted that they are not used in a sense limiting the technical ideas of the contents of the present disclosure.

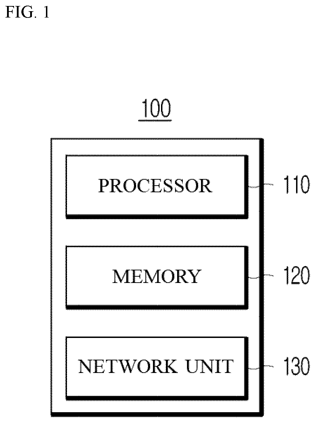

FIG. 1 is a block configuration diagram of a computing device according to an embodiment of the present disclosure.

A computing device 100 according to an embodiment of the present disclosure may be a hardware device or a part of a hardware device that performs comprehensive processing and calculation of data, or may be a software-based computing environment connected to a communication network. For example, the computing device 100 may be a server that performs intensive data processing functions and shares resources, or may be a client that shares resources through interaction with a server. **In** addition, the computing device 100 may be a cloud system in which a plurality of servers and clients interact to comprehensively process data. Since the above description is only one example related to the type of the computing device 100, the type of the computing device 100 may be configured in various ways within a category understandable to those skilled in the art based on the contents of the present disclosure.

Referring to FIG. 1, the computing device 100 according to an embodiment of the present disclosure may include a processor 110, a memory 120, and a network unit 130. However, FIG. 1 is only one example, and the computing device 100 may include other configurations for implementing a computing environment. In addition, only some of the configurations disclosed above may be included in the computing device 100.

The processor 110 according to an embodiment of the present disclosure may be understood as a configuration unit including hardware and/or software for performing computing operations. For example, the processor 110 may read a computer program and perform data processing for machine learning. The processor 110 may process computational processes such as processing input data for machine learning, feature extraction for machine learning, and error calculation based on backpropagation. The processor 110 for performing such data processing may include a central processing unit (CPU), a general purpose graphics processing unit (GPGPU), a tensor processing unit (TPU), an application specific integrated circuit (ASIC), or a field programmable gate array (FPGA), etc. The type of the processor 110 described above is only one example, and thus the type of the processor 110 may be configured in various ways within a category understandable to those skilled in the art based on the contents of the present disclosure.

The processor 110 may filter electrocardiogram data based on the electrocardiogram data and cardiac arrest data of a subject whose electrocardiogram data has been measured. The processor 110 may analyze the electrocardiogram data and the corresponding cardiac arrest data to extract valid data, which may be used for training, from the electrocardiogram data. For example, the processor 110 may analyze whether cardiac arrest occurs, a cardiac arrest occurrence time included in the cardiac arrest data, etc., to filter data that has low learning utility and is difficult to respond to according to the prediction results from the electrocardiogram data. The processor 110 may analyze the extent to which missing values exist in the electrocardiogram data to filter data that does not affect learning or may actually have a negative effect on learning from the electrocardiogram data. In this way, the processor 110 may filter the electrocardiogram data based on information about cardiac arrest or information about missing of the electrocardiogram data itself to select valid data that may be used for learning.

The processor 110 may generate training data for a deep learning model that performs a prediction of a health state based on the filtered electrocardiogram data. The processor 110 may label valid data extracted from the electrocardiogram data and generate training data using the labeled valid data. For example, the processor 110 may label the valid data based on information included in cardiac arrest data corresponding to the valid data. The processor 110 may label information included in the cardiac arrest data, such as whether the cardiac arrest has occurred and the cardiac arrest occurrence time, in the valid data. The processor 110 may determine the labeled valid data as training data for a model that predicts the occurrence of the cardiac arrest.

The processor 110 may train the deep learning model that predicts the health state using the electrocardiogram based on the generated training data. Since the training data includes the input electrocardiogram data and label, when the deep learning model outputs the prediction results of the health state based on the input included in the training data, the processor 110 may compare the output and the label to adjust neural network parameters included in the deep learning model. The processor 110 may train the deep learning model by repeatedly performing the process of adjusting the neural network parameters so that the error between the output and the label is minimized. In this case, the deep learning model may include a neural network that extracts features from the electrocardiogram and a neural network that calculates a prediction value for a health state based on the extracted features. For example, the processor 110 may input valid data labeled with information about cardiac arrest to the deep learning model and output a probability value for the possibility of occurrence of the cardiac arrest (or the possibility of death) in the form of a score between 0 and 100. The processor 110 may calculate an error with a loss function that uses the score and label output by the deep learning model as input variables, and adjust the neural network parameters included in the deep learning model in a direction in which the error decreases. The processor 110 may train the deep learning model to predict cardiac arrest by repeatedly performing this process.

The processor 110 may predict the health state of the subject whose electrocardiogram data has been measured based on newly acquired electrocardiogram data using the trained deep learning model. The processor 110 may input the electrocardiogram data to the trained deep learning model to produce results of predicting the health state of the subject whose electrocardiogram data has been measured. In this case, the results of predicting the health state may include a score expressing how much the overall health has deteriorated, such as how much the possibility of death of the subject whose electrocardiogram data has been measured is. For example, the processor 110 may input the newly acquired electrocardiogram data to the trained deep learning model and output a probability value for the possibility of occurrence of the cardiac arrest (or the possibility of death) in the form of a score between 0 and 100. In this case, it can be understood that 0 denotes a healthy state with a very low possibility of death, and 100 denotes a dangerous state with the highest possibility of death. That is, the processor 110 may use the trained deep learning model to calculate, from electrocardiogram data, a score from which the overall health state of a person may be intuitively understood.

The memory 120 according to the embodiment of the present disclosure may be understood as a configuration unit including hardware and/or software for storing and managing data processed in the computing device 100. That is, the memory 120 may store any type of data generated or determined by the processor 110 and any type of data received by the network unit 130. For example, the memory 120 may include at least one of storage media such as a flash memory type, a hard disk type, a multimedia card micro type, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disc. In addition, the memory 120 may include a database system that controls and manages data in a predetermined system. Since the type of the memory 120 described above is only one example, the type of the memory 120 may be configured in various ways within a category understandable to those skilled in the art based on the content of the present disclosure.

The memory 120 may structure, organize, and manage data necessary for performing an operation, combinations of the data, program codes executable by the processor 110, etc. For example, the memory 120 may store medical data received through the network unit 130 to be described below. The memory 120 may store a program code that operates a neural network model to receive medical data and perform training, a program code that operates the neural network model to receive the medical data and perform inference according to the purpose of use of the computing device 100, and processed data generated as the program codes are executed, etc.

The network unit 130 according to the embodiment of the present disclosure may be understood as a configuration unit that transmits and receives data through any known wired/wireless communication system. For example, the network unit 130 may transmit and receive data using wired or wireless communication systems such as a local area network (LAN), Wideband Code Division Multiple Access (WCDMA), Long Term Evolution (LTE), wireless broadband Internet (WiBro), 5th generation mobile communication (5G), ultra wide-band, ZigBee, radio frequency (RF) communication, a wireless LAN, wireless fidelity, near field communication (NFC), or Bluetooth. Since the above-described communication systems are only examples, the wired and wireless communication system for data transmission and reception of the network unit 130 may be applied in various ways other than the above-described examples.

The network unit 130 may receive data necessary for the processor 110 to perform operations through the wired or wireless communication with any system or any client, etc. In addition, the network unit 130 may transmit data generated by the operation of the processor 110 through the wired or wireless communication with any system, any client, etc. For example, the network unit 130 may receive medical data through communication with a cloud server, clients such as a smart watch, or medical computing devices, etc., that perform tasks such as database within a clinical environment and standardization of medical data. The network unit 130 may transmit output data of the neural network model and intermediate data, processed data, etc., derived from the operation process of the processor 110 through the communication with the above-described database, server, client, computing device, etc.

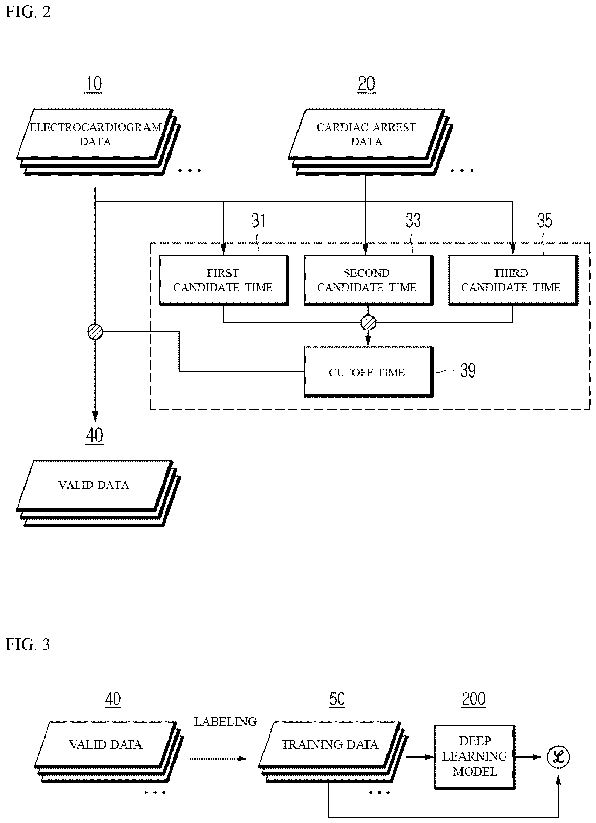

FIG. 2 is a block diagram illustrating a process of extracting valid data from electrocardiogram data according to an embodiment of the present disclosure.

Referring to FIG. 2, a computing device 100 according to an embodiment of the present disclosure may analyze electrocardiogram data 10 and cardiac arrest data 20 to extract valid data 40 from the electrocardiogram data 10. Specifically, the computing device 100 may analyze a cardiac arrest occurrence time recorded in the cardiac arrest data 20 and clinical evidence regarding cardiac arrest to determine a cutoff time 39 for filtering the electrocardiogram data. Then, the computing device 100 may filter electrocardiogram data of a subject who suffers from cardiac arrest within the cutoff time 39 from the electrocardiogram data 10 to extract the valid data 40 from the electrocardiogram data 10. That is, the computing device 100 may calculate the cutoff time 39 based on information regarding cardiac arrest to extract data of a subject who suffers from cardiac arrest within a preset time after the cutoff time 39 as the valid data 40. The electrocardiogram signal has a large range of change before the time of death, and the prediction close to the time of death is not very useful for analysis. Accordingly, the computing device 100 may select the valid data 40 from the electrocardiogram data 10 based on the cutoff time 39 related to the cardiac arrest occurrence time, and prepare data with an analysis value useful for predicting the health state.

For example, the computing device 100 may analyze the cardiac arrest occurrence time recorded in the cardiac arrest data 20 and clinical evidence regarding the cardiac arrest, and derive candidate times for deriving the cutoff time 39. The candidate times may include a first candidate time 31 derived based on clinical determination of a change pattern of the electrocardiogram that appears before the occurrence of the cardiac arrest, a second candidate time 33 clinically determined to be the time at which treatment is possible to perform to prevent the cardiac arrest when the cardiac arrest is predicted, and a third candidate time 35 corresponding to the error between the cardiac arrest occurrence time recorded in the cardiac arrest data and the actual cardiac arrest occurrence time of the subject whose electrocardiogram data has been measured.

The computing device 100 may calculate N minutes, which is determined to have a high probability of cardiac arrest occurrence, a large change, and low accuracy of analysis through the deep learning model, as the first candidate time 31. The electrocardiogram measured may generally have a greater range of changes than a healthy state from N minutes before the occurrence of the cardiac arrest to the time at which the cardiac arrest occurs. **In** addition, even if the corresponding portion is analyzed through the deep learning model, the accuracy of the analysis results on the possibility of cardiac arrest may tend to be low. Therefore, the computing device 100 may calculate the time at which the change pattern of the electrocardiogram is estimated to begin to occur the most greatly based on the clinical determination of the cardiac arrest as the first candidate time 31.

The computing device 100 may calculate, as the second candidate time 33, M minutes, which is the time at which it is determined that the treatment to prevent the cardiac arrest is possible before the time at which it is predicted that the cardiac arrest will occur. Even if the deep learning model predicts a time shorter than M minutes as the predicted time of cardiac arrest, when the time is so short that the treatment to prevent the cardiac arrest is impossible, the meaning of the prediction itself, which is performed for the purpose of preventing the cardiac arrest in advance, is lost. Therefore, the computing device 100 may calculate the clinical time at which the treatment to prevent cardiac arrest is determined to be possible as the second candidate time 33. **In** this case, the time at which the treatment to prevent the cardiac arrest is determined to be possible may be determined based on personal information included in the cardiac arrest data and the clinical evidence regarding cardiac arrest. The computing device 100 may analyze how many minutes from the time at which the cardiac arrest is clinically predicted to occur to perform the treatment to prevent the cardiac arrest in consideration of an age, a gender, etc., of a measurer included in the personal information. **In** addition, the computing device 100 may determine the time derived from the analysis result as the second candidate time 33.

The computing device 100 may calculate L minutes, which is an error between the actual time of cardiac arrest and the time recorded in the cardiac arrest data by medical staff, etc., as the second candidate time 35. **In** this case, the actual time of cardiac arrest may be determined based on the electrocardiogram data. Since the electrocardiogram signal represents cardiac activity, the computing device 100 may determine the time at which the electrocardiogram signal included in the electrocardiogram data does not change as the actual time of cardiac arrest. Even if the heart of a patient who suffered from cardiac arrest actually stops at 12:24, there is a possibility that the medical staff may record that the cardiac arrest occurs at 12:30 according to subjective judgment. **In** this case, although an electrocardiogram measured after 12:26 was an electrocardiogram taken after the cardiac arrest, when viewed in conjunction with cardiac arrest data, it is likely to be mis-analyzed as an electrocardiogram measured 4 minutes before the occurrence of the cardiac arrest. Therefore, in order to prevent the misanalysis due to such errors, the computing device 100 may determine the error between the actual cardiac arrest occurrence time and the cardiac arrest occurrence time included in the cardiac arrest data as the third candidate time 35.

The computing device 100 may compare the sizes of the first candidate time 31, the second candidate time 33, and the third candidate time 35 to determine the cutoff time 39, which is a time with little utility for analysis before the occurrence of the cardiac arrest. The computing device 100 may determine the largest value among the first candidate time 31, the second candidate time 33, or the third candidate time 35 as the cutoff time 39 based on the comparison result. For example, the largest value among the first candidate time 31 that is N minutes, the second candidate time 33 that is M minutes, or the third candidate time 35 that is L minutes may include the remaining time. Therefore, the computing device 100 may determine the largest value among the candidate times 31, 33, and 35 including the remaining time as the cutoff time 39 that is T minutes.

The computing device 100 may exclude data within the cutoff time 39 from the electrocardiogram data 10 and extract the remaining data as the valid data 40. For example, when the cutoff time 39 is determined as T minutes, the computing device 100 may determine data within T minutes from the electrocardiogram data 10 as data with low learning utility. The computing device 100 may determine data after T minutes from the electrocardiogram data 10 as data with high learning utility. **In** addition, the computing device 100 may determine data of a subject who did not suffer from cardiac arrest from the electrocardiogram data 10 as data with high learning utility. **In** addition, the computing device 100 may determine the data with high learning utility from the electrocardiogram data 10 as the valid data 40.

Meanwhile, although not disclosed in FIG. 2, the computing device 100 may analyze missing values of the electrocardiogram data 10 to extract the valid data 40 from the electrocardiogram data 10. The computing device 100 may analyze how many missing values exist based on the derivation itself of the electrocardiogram signal included in the electrocardiogram data 10 or the measurement time. The computing device 100 may filter the electrocardiogram data 10 based on the analysis result to generate the valid data 40. For example, when one or more of N derivations of the electrocardiogram data 10 are missing, the computing device 100 may exclude the electrocardiogram data in which one or more of the N derivations are missing from the valid data 40. When there is a derivation among the N derivations of the electrocardiogram data 10 that has a missing value for a predetermined period of time or longer, the computing device 100 may exclude the electrocardiogram data including the derivation among N derivations that has a missing value for a predetermined period of time or longer from the valid data 40. **In** other words, the computing device 100 may exclude from the valid data 40 the data in which one or more derivations themselves or some of them are missing among the N derivations.

The computing device 100 may use at least one of the method of generating the valid data 40 based on the cutoff time 39 or the method of generating the valid data 40 based on the missing value of the electrocardiogram data 10 according to the user input. For example, when a user who wants to generate training data wants to generate high-purity training data, the computing device 100 may use both a method of generating the valid data 40 based on the cutoff time 39 and a method of generating the valid data 40 based on the missing value of the electrocardiogram data 10 according to the input according to the user's intention to generate the valid data 40.

FIG. 3 is a block diagram illustrating a process of training a deep learning model based on valid data according to an embodiment of the present disclosure.

Referring to FIG. 2 and FIG. 3, the computing device 100 according to an embodiment of the present disclosure may perform labeling on the valid data 40 produced through FIG. 2 described above to generate the training data 50. The computing device 100 may perform labeling based on whether the cardiac arrest occurs and the cardiac arrest occurrence time in the valid data 40 to generate the training data 50. For example, the computing device 100 may label data of a subject who suffered from the cardiac arrest within 24 hours after the cutoff time 39 in the valid data 40 as a cardiac arrest group. **In** addition, the computing device 100 may label data of a subject who did not suffer from cardiac arrest or die within 24 hours after the cutoff time 39 in the valid data 40 as a normal group. **In** other words, the computing device 100 may not classify data whose cardiac arrest occurrence time exceeds 24 hours after the cutoff time 39 into any group in order to reduce the prediction error of the model during the training process. Meanwhile, a figure, which is the above-described 24 hours, is only an example, and the computing device 100 may set the corresponding time in various ways according to the user input.

The computing device 100 may perform the training of the deep learning model 200 based on the training data 50 generated by completing the labeling. **In** this case, the deep learning model 200 may be trained to calculate the probability of cardiac arrest occurring within 24 hours from the time of receiving the electrocardiogram data. For example, the computing device 100 may input the electrocardiogram data included in the training data 50 to the deep learning model 200 to calculate a score representing the probability of occurrence of the cardiac arrest within 24 hours. **In** this case, the score may be a value between 0 and 100 that expresses the probability of occurrence of the cardiac arrest within 24 hours. The computing device 100 may compare the score calculated by the deep learning model 200 with the score corresponding to the label through the loss function to calculate an error. The computing device 100 may adjust the neural network parameters of the deep learning model 200 in the direction in which the error decreases. The computing device 100 may repeat this process and terminate the training of the deep learning model 200 when the error satisfies a minimum criterion. Meanwhile, in FIG. 3, the training of the deep learning model 200 is described based on supervised learning, but the training of the deep learning model 200 may also be performed based on self-supervised learning according to the neural network structure of the deep learning model 200.

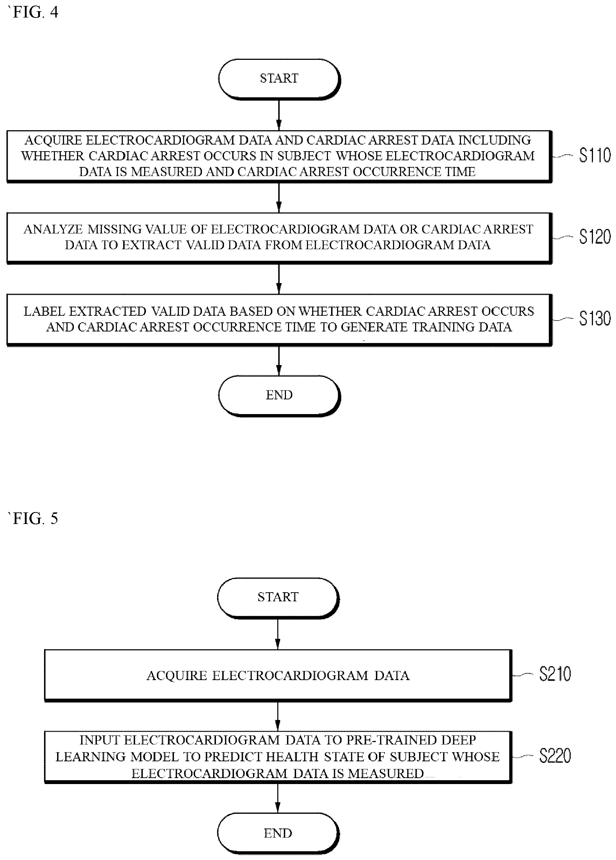

FIG. 4 is a flowchart illustrating a method of predicting a health state using an electrocardiogram according to an embodiment of the present disclosure.

Referring to FIG. 4, the computing device 100 according to an embodiment of the present disclosure may acquire electrocardiogram data, and cardiac arrest data including whether the cardiac arrest occurs in a subject whose electrocardiogram data is measured and the cardiac arrest occurrence time (S110). When the computing device 100 is a server that performs electrocardiogram reading, the computing device 100 may acquire the electrocardiogram data and the corresponding cardiac arrest data through communication with equipment that generates the electrocardiogram data and the cardiac arrest data. When the computing device 100 is a device capable of performing electrocardiogram measurement and recording cardiac arrest-related information, the computing device 100 may generate the electrocardiogram data and the cardiac arrest data based on signals and information input through a measuring unit and an input/output unit.

The computing device 100 may extract the valid data from the electrocardiogram data by analyzing the missing value of the electrocardiogram data or the cardiac arrest data (S120). The computing device 100 may analyze the cardiac arrest occurrence time recorded in the cardiac arrest data and the clinical basis for the cardiac arrest to determine the cutoff time for filtering the electrocardiogram data. The computing device 100 may filter electrocardiogram data of a subject who suffered from the cardiac arrest within the cutoff time among the electrocardiogram data to extract the valid data.

The computing device 100 may label the valid data extracted through operation S120 based on whether the cardiac arrest occurs and the cardiac arrest occurrence time, thereby generating the training data (S130). The computing device 100 may classify data of a subject who suffered from the cardiac arrest and data of a subject who did not suffer from the cardiac arrest or did not die from the valid data. **In** addition, the computing device 100 may label the classified data into two groups.

Although not disclosed in FIG. 4, the computing device 100 may input the training data into the deep learning model to train the deep learning model so that the deep learning model may calculate the probability of occurrence of the cardiac arrest. The description of the training of the deep learning model will be replaced with the description of FIG. 3 described above.

FIG. 5 is a flowchart illustrating a method of predicting a health state using an electrocardiogram according to an alternative embodiment of the present disclosure.

Referring to FIG. 5, the computing device 100 according to an embodiment of the present disclosure may acquire the electrocardiogram data. When the computing device 100 is a server that performs the electrocardiogram reading, the computing device 100 may acquire the electrocardiogram data through communication with equipment such as a wearable device that measures the electrocardiogram data. When the computing device 100 is a device that may perform the electrocardiogram measurement, the computing device 100 may generate the electrocardiogram data based on signals and information input through the measuring unit such as an electrode.

The computing device 100 may predict the health state of a subject whose electrocardiogram data has been measured by inputting the electrocardiogram data to the pre-learned deep learning model (S220). The computing device 100 may calculate a score expressing the degree of possibility that a subject whose electrocardiogram data has been measured will experience the cardiac arrest within 24 hours through the deep learning model to which the electrocardiogram data has been input. The computing device 100 may analyze the degree to which the health state has worsened based on the score and generate a report. When the computing device 100 is the server that performs the electrocardiogram reading, the computing device 100 may share the report through the communication with the equipment such as the wearable device that measures the electrocardiogram data. When the computing device 100 has the input/output unit, the computing device 100 may directly output the report through the input/output unit.

Various embodiments of the present disclosure described above may be combined with additional embodiments and can be modified within a range that may be understood by those skilled in the art in light of the detailed description set forth above. The embodiments of the present disclosure are illustrative in all respects and should be understood as non-limiting. For example, each component described as a single type may be implemented in a distributed manner, and similarly, components described as distributed may be implemented in a combined form. Accordingly, all changes or modifications derived from the meaning, scope, and equivalent concept of the claims of the present disclosure should be construed as being included in the scope of the present disclosure.

## Claims

1. A method of predicting a health state using an electrocardiogram, performed by a computing device including at least one processor, comprising:
acquiring electrocardiogram data and cardiac arrest data including whether cardiac arrest occurs in a subject whose electrocardiogram data is measured and a cardiac arrest occurrence time of the subject;
analyzing a missing value of the electrocardiogram data or the cardiac arrest data to extract valid data from the electrocardiogram data; and
labeling the extracted valid data based on whether the cardiac arrest occurs and the cardiac arrest occurrence time to generate training data.

2. The method of claim 1, wherein the analyzing of the missing value of the electrocardiogram data or the cardiac arrest data to extract the valid data from the electrocardiogram data includes:
analyzing the cardiac arrest occurrence time recorded in the cardiac arrest data and clinical evidence regarding cardiac arrest to determine a cutoff time for filtering the electrocardiogram data; and
filtering the electrocardiogram data of the subject who suffers from the cardiac arrest within the cutoff time among the acquired electrocardiogram data to extract the valid data.

3. The method of claim 2, wherein the analyzing of the cardiac arrest occurrence time recorded in the cardiac arrest data and the clinical evidence regarding the cardiac arrest to determine the cutoff time for filtering the electrocardiogram data includes determining, as the cutoff time, one of a first candidate time derived based on clinical determination on a pattern of change in the electrocardiogram that occurs before the cardiac arrest occurs, a second candidate time clinically determined as a time at which treatment is possible to perform to prevent the cardiac arrest when the cardiac arrest is predicted to occur, and a third candidate time corresponding to an error between the cardiac arrest occurrence time recorded in the cardiac arrest data and an actual cardiac arrest occurrence time of the subject whose electrocardiogram data is measured.

4. The method of claim 3, wherein the cutoff time is determined as the largest value among the first candidate time, the second candidate time, or the third candidate time.

5. The method of claim 4, wherein the labeling of the extracted valid data based on whether the cardiac arrest occurs and the cardiac arrest occurrence time to generate the training data includes: labeling, as a cardiac arrest group, data of the subject who suffers from the cardiac arrest within a preset time after the cutoff time in the valid data; and labeling, as a normal group, data of the subject who does not suffer from cardiac arrest or die within the preset time after the cutoff time in the valid data.

6. The method of claim 1, wherein the analyzing of the missing value of the electrocardiogram data or the cardiac arrest data to extract the valid data from the electrocardiogram data includes excluding the electrocardiogram data in which one or more of N derivations of the electrocardiogram data themselves are missing from the valid data when one or more of the N derivations of the electrocardiogram data themselves are missing.

7. The method of claim 1, wherein the analyzing of the missing value of the electrocardiogram data or the cardiac arrest data to extract the valid data from the electrocardiogram data includes excluding, from the valid data, the electrocardiogram data including a derivation in which the missing value exists for a predetermined period of time or longer among the N derivations when there is the derivation in which the missing value exists for the predetermined period of time or longer among the N derivations of the electrocardiogram data.

8. The method of claim 1, further comprising inputting the training data to a deep learning model to train the deep learning model so that the deep learning model calculates the possibility of occurrence of the cardiac arrest.

9. A method of predicting a health state using an electrocardiogram, performed by a computing device including at least one processor, comprising:
acquiring first electrocardiogram data; and
inputting the first electrocardiogram data to a pre-trained deep learning model to predict the health state of a subject whose electrocardiogram data is measured,
wherein the deep learning model is trained based on training data generated by labeling valid data extracted from second electrocardiogram data based on whether cardiac arrest occurs and a cardiac arrest occurrence time, and
the valid data is extracted from the second electrocardiogram data based on results of analyzing a missing value of the second electrocardiogram data or cardiac arrest data including whether the cardiac arrest occurs in a subject whose second electrocardiogram data is measured and a cardiac arrest occurrence time of the subject.

10. The method of claim 9, wherein the prediction result derived by inputting the first electrocardiogram to the pre-trained deep learning model includes a score value indicating the health state of the subject whose electrocardiogram data is measured, and
the score value indicates a healthy state as it approaches a first threshold value, and indicates an unhealthy state as it approaches a second threshold value.

11. A computer program that is stored in a computer-readable storage medium and performs operations for predicting a health state using an electrocardiogram when executed on one or more processors, wherein the operations include:
acquiring electrocardiogram data and cardiac arrest data including whether cardiac arrest occurs in a subject whose electrocardiogram data is measured and a cardiac arrest occurrence time of the subject;
analyzing a missing value of the electrocardiogram data or the cardiac arrest data to extract valid data from the electrocardiogram data; and
labeling the extracted valid data based on whether the cardiac arrest occurs and the cardiac arrest occurrence time to generate training data.

12. A computing device for predicting a health state using an electrocardiogram, comprising:
a processor including at least one core;
a memory including program codes executable in the processor; and
a network unit configured to acquire electrocardiogram data and cardiac arrest data including whether cardiac arrest occurs in a subject whose electrocardiogram data is measured and a cardiac arrest occurrence time of the subject,
wherein the processor analyzes a missing value of the electrocardiogram data or the cardiac arrest data to extract valid data from the electrocardiogram data, and
labels the extracted valid data based on whether the cardiac arrest occurs and the cardiac arrest occurrence time to generate training data.
